# EUROPÄISCHE PATENTSCHRIFT

(11) **EP 0 509 340 B1**
(45) Veröffentlichungstag und Bekanntmachung des Hinweises auf die Patenterteilung: **22.01.1997**
(21) Anmeldenummer: 92105744.4
(22) Anmeldetag: 03.04.1992
(51) Int. Cl.: C12M 1/10

(54) **Horizontaler rotierender Schüttbettreaktor für biotechnologische Prozesse**
Horizontal rotating reactor with moving bed for biotechnoligical processes
Réacteur rotatif horizontal à lit agité pour procédés biotechnologiques

(30) Priorität: 15.04.1991 DE 4112236
(43) Veröffentlichungstag der Anmeldung: 21.10.1992
(73) Patentinhaber: FORSCHUNGSZENTRUM JÜLICH GMBH, 52425 Jülich (DE); AMBS & CO KG, D-79305 Emmendingen (DE)
(72) Erfinder: Overath, Horst, W-5170 Jülich (DE); Kern, Rolf, W-7830 Emmendingen (DE)

(56) Entgegenhaltungen:
- EP-A- 0 237 039
- EP-A- 0 393 651
- WO-A-86/04085
- WO-A-89/00612
- JOURNAL OF THE FACULTY OF ENGINEERING. Bd. 38, Nr. 1, 1985, TOKYO, JAPAN. S. 1-7; S. FURUSAKI et al.

## Beschreibung

Die Erfindung bezieht sich auf einen horizontalen Schüttbettreaktor für biotechnologische Prozesse mit rotierender Trommel, von deren Innenwand Längsrippen in die Schüttung hinein vorspringen und die mit Flüssigkeitszu- und -ablauf sowie mit Einrichtungen für die Gasabgabe versehen ist.

Für die Durchführung biotechnologischer Prozesse, insbesondere von Fermentationsprozessen, die mit Hilfe von Mikroorganismen ablaufen, sind unter anderem Reaktoren bekannt, bei denen die Mikroorganismen als Bewuchs auf einem gekörnten Trägermaterial vorliegen. Weit verbreitet sind dabei senkrechte Rohrreaktoren mit einem bakterienbesiedelten Schüttkörperbett, durch das die umzusetzende Flüssigkeit hindurchgeleitet wird.

Desweiteren ist von Furusaki et al. (in: Journal of the faculty ofEngineering, Bd. 38, Nr. 1, 1985, Seiten 1 bis 7) auch ein rotierender, horizontaler Reaktor für die Ethanol-produktion mit immobilisierten Hefezellen beschrieben. Dieser Reaktor ist durch Querwände in mehrere Kammern unterteilt, wobei jede Kammer ein Sieb aufweist, das zum einen die Feststoffe zurückhält und durch das zum anderen die Prozeßflüssigkeit in die jeweils nächste Kammer gelangt.

Üblicherweise laufen biotechnologische Prozesse unter Gasentwicklung und Biomasseproduktion ab, weshalb die Effizienz der Umsetzung in Schüttkörperbetten, bedingt durch austauschbehindernde Gasfilme oder Blasen, vermindert ist und auf lange Sicht Verstopfungsprobleme durch überschüssige Biomasseproduktion und Kanalbildungen auftreten.

Von der Anmelderin wurde daher bereits ein rotierender Schüttbettreaktor entwickelt (EP-OS 0 237 039), bei dem ein allgemein horizontales, mit Mikroorganismen besiedeltes Schüttkörperbett in einem Rohrreaktor unter Freilassung eines Restvolumensenthalten ist, der ständig oder intermittierend in langsame Dreh- oder Schaukelbewegungen versetzt wird, die eine Umwälzung des Bettmaterials herbeiführen. Dabei wird zum einen die Ablösung von Gasblasen vom Schüttkörper favorisiert und zum anderen eine Verstopfung durch überschüssige Biomassebildung verhindert. Ein übermäßiger Austrag von Biomasse oder gar Abrieb des Schüttkörpermaterials ist in Anbetracht der relativ geringen Rotationsgeschwindigkeit von maximal 10 Upm zurückgedrängt.

Ein solcher rotierender Schüttbettreaktor soll ggfs. vom Rohrmantel nach innen vorspringende Längsrippen zur Verbesserung der Kornmitnahme aufweisen.

Gemäß EP-A-O- 393 651 soll die Arbeitsweise eines solchen rotierenden Schüttbettreaktors mit Längsrippen dadurch verbessert werden, daß von diesen Ringblechabschnitte in die Schüttung hinein vorstehen, wodurch Kurzschlußströmungen vermieden werden sollen.

Auch bei diesem Reaktorkonzept ergeben sich jedoch noch Minderungen in der Abbauleistung. Ziel der Erfindung ist daher eine weitere strömungstechnische Optimierung des rotierenden Schüttbettreaktors.

Der zu diesem Zweck entwickelte erfindungsgemäße Reaktor der eingangs genannten Art ist im wesentlichen gekennzeichnet durch eine Unterteilung der Trommel in Längsrichtung in zumindest zwei Kammern durch Querwände mit den Schüttkörper zurückhaltender Sieböffnung für den Flüssigkeitdurchtritt und durch eine kammerweise Unterteilung der Längsrippen, die von Kammer zu Kammer winkelversetzt angebracht sind.

Durch die Aufteilung des Rohrreaktors in Einzelkammern mit Flüssigkeitsdurchlaß durch die jeweiligen Trennwände wird zum einen die Möglichkeit eröffnet, unterschiedliche Schüttkörper in (den) einzelnen Kammern zwischen Flüssigkeitseinlaß und -auslaß vorzusehen, und es wird ferner verhindert, daß bei längerem Betrieb eine Wanderung von Schüttkörper zum Flüssigkeitsauslaß hin stattfindet. Die Querwände erzwingen zudem eine bessere strömungsmäßige Ausnutzung der gesamten Kammerfüllung und die von Kammer zu Kammer versetzten Längsrippen wirken zusätzlich als Raumausnutzung verbessernde Leitelemente. Sie tragen dazu bei, die Radialbewegung der Körner zu verbessern und damit eine ausreichende radiale Durchmischung der Schüttung zu gewährleisten.

Die flüssigkeitsdurchlässigen Sieböffnungen in den als Kammerabschluß wirkenden Querwänden sollen den Schüttkörper zurückhalten und sie haben bereits von daher geringe Öffnungsquerschnitte, die zu einer gewissen Angleichung der Strömungswiderstände von Schüttkörperfüllung und Sieböffnung führen, so daß ein die Durchdringung der Kammerfüllung vermeidender unmittelbarer Flüssigkeitsdurchgang über die Sieböffnungen vom Einlaß zum Auslaß der Trommel verhindert wird.

Zweckmäßig wäre z.B. ein in die Kammern vorragender Siebkörper, wie etwa ein rohrförmiger Lochkorb, vorzugsweise werden diese Sieböffnungen jedoch durch rohrstutzenförmige Spaltsiebe mit stirnseitigem Abschluß gebildet, die im wesentlichen durch einen Stabkranz aus Rundstäben oder Vierkantstäben oder auch von Stäben mit anderem Querschnitt gebildet werden. Der Durchmesser der Stäbe (oder ggfs. Rohre) liegt im allgemeinen zwischen 5 und 15 mm. Besonders zweckmäßig sind gleichsinnig angeordnete Stäbe mit dreieckigem oder trapezförmigen Querschnitt.

Die nur flüssigkeitsdurchlässige Unterteilung des Rohrreaktors in einzelne Kammern gestattet einen von Kammer zu Kammer unterschiedlichen Einsatz von Schüttkörpern und zwar sowohl hinsichtlich der Korngröße und Kornbeschaffenheit, wie insbesondere Porosität und des Kornmaterials als auch ggfs. der Besiedelung mit Mikroorganismen. So kann beispielsweise spezielles spurenelementhaltiges Material insbesondere in den zulaufseitigen Kammern vorgesehen werden oder in der letzten Kammer Dolomit enthalten sein zur abschließenden Aufhärtung der aus dem Rohrreaktor abgegebenen Flüssigkeit.

Gemäß einer bevorzugten Ausgestaltung für größere Reaktoren sind jeweils in Kammermitte scheibenförmige Umlenkbleche vorgesehen, die als Flüssigkeitsdurchlaß von einer Kammerhälfte zur anderen einen Ringspalt zum Trommelmantel hin freilassen, dessen Breite vorzugsweise bei 5 - 10 % des Trommeldurchmessers liegt. Bei dieser Ausgestaltung sind die Spaltsiebstutzen zweckmäßigerweise jeweils bis zum Umlenkblech durchgezogen, wobei dann aufgrund der größeren Länge der Spaltsiebstutzen ihr Durchmesser vermindert sein kann.

Selbstverständlich braucht der Querschnitt der Spaltsiebstutzen nicht kreisringförmig zu sein, obzwar dies weitgehend zweckmäßig erscheint. Im in sich geschlossenen Innenraum der Spaltsiebe sind vorzugsweise Füllkörper in Form eines scharfkantigen Granulats aus Inertmaterial enthalten, die für eine Selbstreinigung der Sieböffnungen bei der Rotation sorgen, was insbesondere bei lebhafter Biomassevermehrung wichtig ist. Als zweckmäßig hat sich eine etwa 20 %ige Füllung mit Edelstahl-Grobkorn bzw. -Schotter (z.B. alte Schrauben oder Muttern) erwiesen.

Weitere Besonderheiten der Erfindung gehen aus den Patentansprüchen, sowie aus der nachfolgenden Beschreibung von Ausführungsbeispielen hervor. Dabei wird Bezug genommen auf die angefügten Zeichnungen; Es zeigen schematisch:
- Figur 1 und 5: zwei Varianten des rotierenden Schüttbettreaktors;
- Figur 2: ein in die Kammerbegrenzungswand eingesetztes Spaltsieb in vergrößerter Form;
- Figur 3: den Querschnitt eines füllkörperhaltigen Spaltsiebes und
- Figur 4: eine zu Figur 3 abgewandelte Spaltsiebvariante innerhalb des Reaktors.

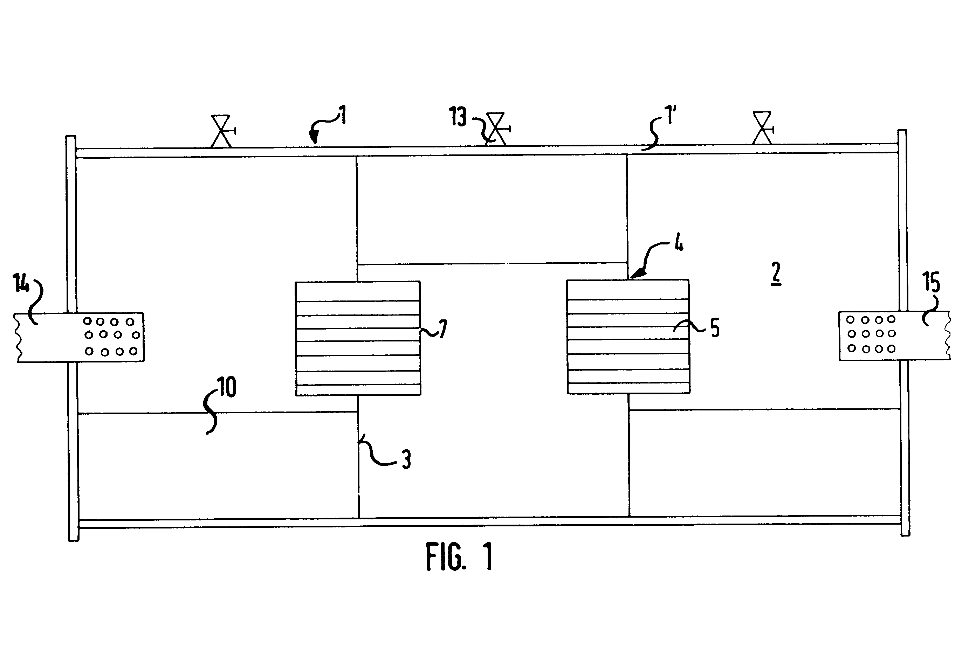

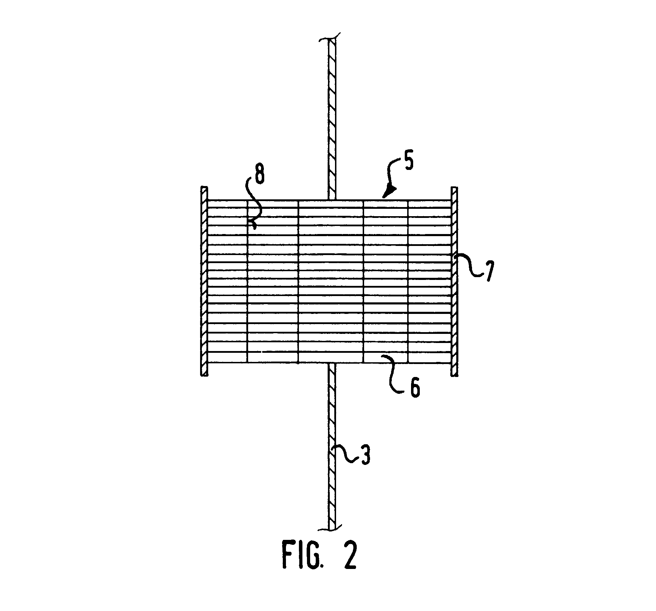

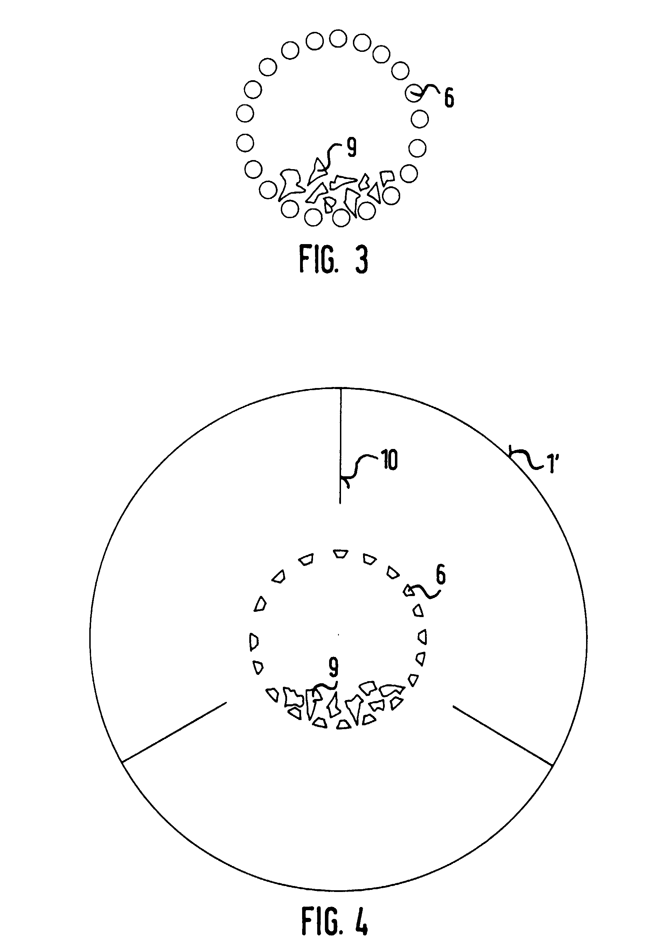

Gemäß Figur 1 ist die drehbare Reaktortrommel 1 in ihrem Innern mit nicht näher dargestelltem Schüttkörper 2 als Träger für Mikroorganismen weitgehend gefüllt (bis zu 95 %), und sie wird durch Querwände 3 in Kammern unterteilt, die einen Flüssigkeitsdurchlaß 4 aufweisen, der im gezeigten Fall durch stutzenförmige Spaltsiebe 5 gebildet wird, die, wie in Figur 2 bis 4 gezeigt wird, durch einen Kranz eng benachbarter Stäbe 6 mit jeweils stirnseitigem Deckel 7 gebildet werden und abgedichtet in eine Öffnung der Kammerwand 3 eingesetzt sind. Mit 8 sind Versteifungsringe der Spaltsiebe 5 angedeutet, die eine Füllung 9 aus inertem scharfkantigen Material, wie insbesondere Edelstahlschotter enthalten, die bei der Rotation der Anordnung für eine "Selbstreinigung" des Spaltsiebes von anwachsender Biomasse sorgt.

Die Längsrippen 10 sind von Kammer zu Kammer gegeneinander versetzt und zwar vorzugsweise so, daß die Längsrippe einer nachfolgenden Kammer in der Mitte zwischen zwei Längsrippen der vorhergehenden Kammer vorgesehen ist.

Die Längsrippen 10 ragen radial vom Trommelmantel 1' weg nach innen und reichen bis etwa ein Drittel des Trommelinnendurchmessers. Zweckmäßig sind Querausdehnungen der Rippen 10 zwischen 10 und 30 % des Kammerdurchmessers und die Zahl der Rippen wird üblicherweise je nach Größe des Reaktors bei 3 oder 4 liegen.

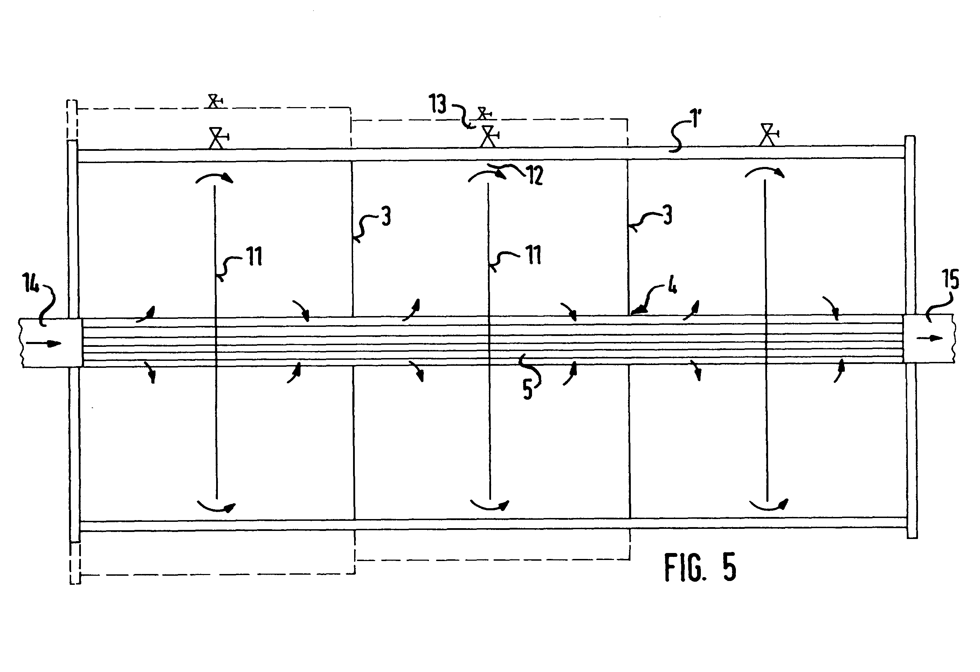

Der Reaktor gemäß Figur 5 enthält in jeder Kammer ein praktisch mittig angeordnetes Umlenkblech 11, das zum Trommelmantel 1' hin einen ringförmigen Durchlaßspalt 12 bildet. Die Anordnung gemäß Figur 5 enthält ebenfalls Längsrippen, die jedoch in der Zeichnung nicht dargestellt sind.

Sowohl in Figur 1 als auch in Figur 5 sind Gasauslaßventile 13 je Kammer (wie schematisch angedeutet) vorgesehen, die zweckmäßigerweise fluchtend vorgesehen werden, um die Einrichtungen zur Betätigung derselben zu vereinfachen. Mit einem nicht dargestellten Niveaugefäß wird der Flüssigkeitsspiegel in den Kammern eingestellt. Er liegt zweckmäßigerweise etwas unter der Oberfläche der Schüttung, die bis zu 95 % des jeweiligen Kammervolumens einnimmt.

In Figur 5 ist eine Abwandlung gestrichelt angedeutet: Danach ist der Durchmesser der Kammern vom linksseitigen Flüssigkeitseinlaß 14 bis zum rechten Flüssigkeitsauslaß 15 abgestuft verringert. Auf diese Weise kann dem zunehmenden Druckabfall der Flüssigkeit vom Reaktoreinlaß bis zum Auslaß und damit je Kammer sinkenden Flüssigkeitsspiegel Rechnung getragen werden.

Eine Realisierung des in Figur 1 gezeigten Reaktors hat einen Trommeldurchmesser von etwa 1 m bei einer Länge von 2,5 m. Die Kammern sind mit etwa kugelförmigen Blähtonkörnern von 4 bis 8 mm Korngröße zu 90 - 95 % gefüllt, deren Umwälzung durch kontinuierliche Rotation des Reaktors mit 0,1 Upm erreicht wird. Der Flüssigkeits-Zu- und -Ablauf erfolgt, wie angedeutet, über Lochstutzen, die bis zu etwa einem Drittel in die jeweilige Kammer hineinragen.

Die Rotation des Reaktors richtet sich nach dem Reaktordurchmesser und liegt bei einem Trommeldurchmesser von 1 m, zweckmäßigerweise im Bereich von 0,05 bis 0,5 Upm. Bei größeren Durchmessern wird die Umdrehungsgeschwindigkeit verringert. Eine Aussage über ggfs. zu hohe Drehgeschwindigkeiten erhält man durch Beobachtung der Trübung am Flüssigkeitsauslaß, die vorzugsweise gering gehalten wird, um Nachbehandlungen der Flüssigkeit möglichst weitgehend zu verringern, insbesondere wenn für diese, wie z.B. bei der Denitrifikation von Wasser, Verbrauchsqualität angestrebt wird.

Bei der Anwendung der vorstehend beschriebenen Vorrichtung zur Denitrifikation von Trinkwasser unter Zusatz von Äthanol konnte bei einem Durchsatz von 5 m³/h eine praktisch vollständige Nitratbeseitigung mit stöchiometrischer Substratzugabe erreicht werden.

## Patentansprüche

1. Horizontaler Schüttbettreaktor für biotechnologische Prozesse mit rotierender Trommel, von deren Innenwand Längsrippen in die Schüttung hinein vorspringen und die mit Flüssigkeitszu- und -ablauf sowie mit Einrichtungen für die Gasabgabe versehen ist,
**gekennzeichnet durch**
eine Unterteilung der Trommel (1) in Längsrichtung in zumindest zwei Kammern durch Querwände (3) mit den Schüttkörper (2) zurückhaltender Sieböffnung (4) für den Flüssigkeitsdurchtritt und durch eine kammerweise Unterteilung der Längsrippen (10), die von Kammer zu Kammer winkelversetzt angebracht sind.

2. Schüttbettreaktor nach Anspruch 1,
**dadurch gekennzeichnet**,
daß die Sieböffnung (4) der Querwände (3) durch beidseits in die angrenzenden Kammern hineinragende, rohrstutzenförmige Spaltsiebe (5) mit stirnseitigem Abschluß (7) gebildet sind, die eine Teilfüllung (9) aus abriebfesten, insbesondere scharfkantigen Füllkörpern aufweisen.

3. Schüttbettreaktor nach Anspruch 2,
**gekennzeichnet durch**
Umlenkbleche (11) mit Ringspalt (12) zum Trommelmantel (1') hin in der Kammermitte, bis zu denen die Spaltsiebe (5) reichen

4. Schüttbettreaktor nach Anspruch 3,
**gekennzeichnet durch**
eine Ringspaltbreite von 5 - 10 % des Trommeldurchmessers.

5. Schüttbettreaktor nach einem der vorangehenden Ansprüche,
**dadurch gekennzeichnet**,
daß die Spaltsiebe (5) aus Stäben (6) von dreieckigem oder trapezförmigem Querschnitt gebildet sind.

6. Schüttbettreaktor nach einem der Ansprüche 2 bis 5,
**gekennzeichnet durch**
eine 20%ige Füllung (9) der Spaltsiebe (5) mit scharfkantigen Granulat aus Inertmaterial.

7. Schüttbettreaktor nach einem der vorangehenden Ansprüche,
**gekennzeichnet durch**
drei Kammern mit je vier von Kammer zu Kammer um 45° gegeneinander versetzten Längsrippen (10) einer Breite von 15 bis 30 % des Trommeldurchmessers, durch eine 2 bis 15 % Freiraum lassende Schüttkörperfüllung (2) mit 4 - 8 mm Korngröße sowie durch einen Rotationsantrieb für Drehgeschwindigkeiten von 0,1 - 1 Upm.

8. Schüttbettreaktor nach einem der vorangehenden Ansprüche,
**gekennzeichnet durch**
eine zum Flüssigkeitsauslaß (15) hin abnehmende Kammergröße.

9. Schüttbettreaktor nach einem der vorangehenden Ansprüche,
**gekennzeichnet durch**
unterschiedliche Schüttkörper in den einzelnen Kammern.

10. Schüttbettreaktor nach Anspruch 9,
**dadurch gekennzeichnet**,
daß sich die Schüttkörper der einzelnen Kammern durch Korngröße, Beschaffenheit, Porosität, Material und/oder Besiedelung voneinander unterscheiden.

## Claims

1. Horizontal granular-bed reactor for biotechnological processes, having a rotating drum from whose internal wall longitudinal ribs project into the bed and which is provided with liquid inlet and liquid outlet and also with devices for the discharge of gas, characterized by a subdivision of the drum (1) in the longitudinal direction into at least two chambers by transverse walls (3) having a screen opening (4) for the passage of liquid, which screen opening retains the granular bodies (2), and by a chamber-wise subdivision of the longitudinal ribs (10), which are mounted with an angular offset from chamber to chamber.

2. Granular-bed reactor according to Claim 1, characterized in that the screen opening (4) of the transverse walls (3) is formed by slotted screens (5) in pipe-socket form which project on both sides into the adjacent chambers and have end-face termination (7) and which have a partial filling (9) of abrasion-resistant, in particular, sharp-edged packing bodies.

3. Granular-bed reactor according to Claim 2, characterized by deflection plates (11) having an annular gap (12) with respect to the drum casing (1') in the centre of the chamber, up to which deflection plates the slotted screens (5) extend.

4. Granular-bed reactor according to Claim 3, characterized by an annular-gap width of 5 - 10% of the drum diameter.

5. Granular-bed reactor according to one of the preceding claims, characterized in that the slotted screens (5) are formed from rods (6) of triangular or trapezoidal cross-section.

6. Granular-bed reactor according to one of Claims 2 to 5, characterized by a 20% packing (9) of the slotted screens (5) with sharp-edged granules of inert material.

7. Granular-bed reactor according to one of the preceding claims, characterized by three chambers each having four longitudinal ribs (10) having a width of 15 to 30% of the drum diameter and offset by 45° with respect to one another from chamber to chamber, by a granular-body packing (2) having a particle size of 4 - 8 mm and leaving a free space of 2 to 15%, and by a rotary drive for rotary speeds of 0.1 - 1 rpm.

8. Granular-bed reactor according to one of the preceding claims, characterized by a chamber size which decreases towards the liquid outlet (15).

9. Granular-bed reactor according to one of the preceding claims, characterized by different granular bodies in the individual chambers.

10. Granular-bed reactor according to Claim 9, characterized in that the granular bodies of the individual chambers differ from one another in particle size, nature, porosity, material and/or colonization.

## Revendications

1. Réacteur horizontal à lit circulant pour procédés biotechnologiques, comportant un tambour rotatif avec des nervures longitudinales qui avancent dans le déversement à partir de la paroi interne dudit tambour, lequel est pourvu d'une arrivée et d'une sortie de liquide, ainsi que des dispositifs pour le dégagement de gaz, caractérisé par une subdivision du tambour (1), dans la direction longitudinale, en au moins deux chambres, au moyen de parois transversales (3) avec une ouverture de tamis (4), retenant le corps en vrac (2), pour le passage du liquide, et par une subdivision en chambres des nervures longitudinales (10) qui sont disposées décalées de façon angulaire d'une chambre à l'autre.

2. Réacteur à lit circulant selon la revendication 1, caractérisé en ce que l'ouverture de tamis (4) des parois transversales (3) est formée de tamis à fentes (5) en forme de tubulure, dépassant des deux côtés à l'intérieur des chambres contiguës, avec une fermeture (7) du côté frontal, qui présentent une charge partielle (9) formée d'éléments de charge résistants à l'usure, en particulier à arêtes vives.

3. Réacteur à lit circulant selon la revendication 2, caractérisé par des chicanes (11) avec une fente annulaire (12), qui vont de l'enveloppe du tambour (1') au centre de la chambre jusqu'à ce qu'elles atteignent le tamis à fentes (5).

4. Réacteur à lit circulant selon la revendication 3, caractérisé par une largeur de fente annulaire valant de 5 à 10 % du diamètre du tambour.

5. Réacteur à lit circulant selon l'une quelconque des revendications précédentes, caractérisé en ce que les tamis à fentes (5) sont formés de barres (6) ayant une section transversale triangulaire ou trapézoïdale.

6. Réacteur à lit circulant selon l'une des revendications 2 à 5, caractérisé par une charge (9) de 20 % des tamis à fentes (5) avec des granules à arêtes vives formés d'un matériau inerte.

7. Réacteur à lit circulant selon l'une des revendications précédentes, caractérisé par trois chambres contenant chacune quatre nervures longitudinales (10), décalées de 45° entre elles d'une chambre à l'autre, d'une largeur valant de 15 à 30 % du diamètre du tambour, par une charge de corps en vrac (2) ayant une dimension de grains de 4 à 8 mm et laissant un espace libre de 2 à 15 %, ainsi que par une commande de rotation pour des vitesses de rotation de 0,1 à 1 tr/min.

8. Réacteur à lit circulant selon l'une des revendications précédentes, caractérisé par une dimension de chambre qui diminue en direction de la sortie (15) pour le liquide.

9. Réacteur à lit circulant selon l'une des revendications précédentes, caractérisé par des corps en vrac différents dans les chambres individuelles.

10. Réacteur à lit circulant selon la revendication 9, caractérisé en ce que les corps en vrac des différentes chambres se différencient les uns des autres par leur dimension de grains, leur nature, leur porosité, leur matériau et/ou leur population.
